# EUROPEAN PATENT SPECIFICATION

(11) **EP 2 491 848 B1**
(45) Date of publication and mention of the grant of the patent: **15.01.2014**
(21) Application number: 12153364.0
(22) Date of filing: 31.01.2012
(51) Int. Cl.: A61B 1/00, A61B 1/12

(54) **Endoscope having end nozzle**
Endoskop mit Enddüse
Endoscope doté d'une buse à son extrémité

(30) Priority: 28.02.2011 JP 2011042185
(43) Date of publication of application: 29.08.2012
(73) Proprietor: Fujifilm Corporation, Minato-ku Tokyo (JP)
(72) Inventor: Ohki, Tomohiro, Kanagawa (JP); Maruhashi, Atsushi, Kanagawa (JP); Matsunaga, Jun, Kanagawa (JP)
(74) Representative: Klunker . Schmitt-Nilson . Hirsch

(56) References cited:
- JP-A- 5 207 962
- JP-A- 2009 279 291
- JP-U- 56 113 503
- JP-U- 62 192 701

## Description

### BACKGROUND OF THE INVENTION

### 1. Field of the Invention

The present invention relates to an endoscope having an end nozzle. More particularly, the present invention relates to an endoscope having an end nozzle, in which an imaging window area is washed, and plural lighting window areas can be protected from sticking of dirt or other unwanted fluid.

### 2. Description Related to the Prior Art

An endoscope includes a section of an elongated tube, a head assembly, an imaging window area, lighting window areas, and an end nozzle device. The head assembly is entered in a body cavity of a body for imaging. The imaging window area, the lighting window areas and the end nozzle device are disposed in the head assembly. The imaging window area receives image light from the body. The lighting window areas emit light to the body. The end nozzle device ejects washing fluid such as water and air to the imaging window area. A first surface is defined on a distal side of the elongated tube, and is perpendicular to an axial direction of the elongated tube. The imaging window area and the end nozzle device are disposed on the first surface. The lighting window areas are arranged so that the imaging window area is surrounded by the lighting window areas.

In general, a window surface of the imaging window area is kept flush with the first surface of the elongated tube. Body fluid or dirt in the body is likely to stick on the window surface of the imaging window area. Use of the end nozzle device is disclosed in JP-B 3583542 (corresponding to JP-A 9-238893), U.S. Pat. No. 6,409, 657 (corresponding to JP-A 2000-279370), and EP-A 2011428 (corresponding to JP-A 2007-289355 and U.S. Pat. Pub. No. 2009/048,490). A nozzle opening in the end nozzle device ejects water to wash away the dirt from the imaging window area. Also, air is ejected through the nozzle opening to blow away droplets of water from the window surface of the imaging window area.

It is likely that the body fluid or dirt from the body is present on the window surface of the lighting window areas in addition to the window surface of the imaging window area. The lighting window areas are washed sometimes but less frequently than the imaging window area is. If the dirt stuck on the window surface of the lighting window areas becomes increased, a light amount of light applied to the body decreases to cause difficulty in the imaging. If a light source apparatus having a light adjuster is used, the light adjuster automatically operates to increase the light amount of the light in response to a decrease in the brightness in the body according to the dirt of the lighting window areas in the endoscope. Problems arise in that walls of the head assembly of the endoscope may be degraded by overheat, and that the dirt may be dried and stuck on the lighting window areas in an unremovable manner.

However, JP-B 3583542 (corresponding to JP-A 9-238893) and U.S. Pat. No. 6,409,657 (corresponding to JP-A 2000-279370) disclose only washing of the imaging window area. The washing fluid ejected by the end nozzle device impinges on the imaging window area to remove the dirt, but does not impinge on the lighting window areas. No removal of the dirt from the lighting window areas is suggested. In EP-A 2011428 (corresponding to JP-A 2007-289355 and U.S. Pat. Pub. No. 2009/048,490), one of the lighting window areas is disposed near to a position in a path direction of the end nozzle device, and may be washed. However, remaining ones of the lighting window areas are disposed away from the path direction of the washing fluid from the end nozzle device.

JP-A 2009-279 291 discloses another imaging window flushing arrangement.

### SUMMARY OF THE INVENTION

In view of the foregoing problems, an object of the present invention is to provide an endoscope having an end nozzle, in which an imaging window area is washed, and plural lighting window areas can be protected from sticking of dirt or other unwanted fluid.

In order to achieve the above and other objects and advantages of this invention, an endoscope having a section of an elongated tube for entry in a body cavity is provided. A first surface is formed at a distal end of a head assembly included in the elongated tube, and disposed to extend crosswise to an axial direction of the elongated tube. A recess is formed in the head assembly at a lower level than the first surface. At least one lighting window area is disposed in the recess, for applying light to an object in the body cavity. An imaging window area is disposed in the first surface, for receiving light from the object. An end nozzle is disposed on the first surface, for washing the imaging window area by ejecting washing fluid thereto. A distal projection is formed on the head assembly at a higher level than the first surface, for receiving the washing fluid from the imaging window area.

A surface height of the imaging window area is larger than a height of the first surface.

The recess is annular and tapered, and disposed to surround the lighting window area.

The recess extends continuously with the distal projection.

In one preferred embodiment, the distal projection extends at least partially along an outer surface of the head assembly. The recess is disposed between a periphery of the imaging window area and the distal projection, is in an arcuate shape, and has a second surface where the lighting window area is disposed.

Furthermore, an inclined guide surface is disposed to extend continuously between the first and second surfaces.

The guide surface is disposed along a periphery of the imaging window area.

Also, an endoscope includes a section of an elongated tube, having a head assembly, for entry in a body cavity in a body. A first surface is disposed at a distal end of the head assembly in an axial direction of the elongated tube. Plural lighting window areas are formed in the first surface, for applying light to an object in the body cavity. An imaging window area is formed in the first surface, for receiving object light from the object. An end nozzle device is disposed on the head assembly, for washing the imaging window area by blowing washing fluid thereto. A distal projection is disposed to project from the first surface in the axial direction, for receiving the washing fluid ejected from the end nozzle device toward the imaging window area.

Furthermore, a recess is formed in the first surface, disposed on a periphery of the lighting window areas, for directing the washing fluid to the lighting window areas.

The end nozzle device includes a flow conduit, disposed in the head assembly, for supplying the washing fluid in the axial direction. A nozzle spout is formed at a distal end of the flow conduit, for directing the washing fluid from the flow conduit toward the imaging window area on the first surface.

The recess is disposed annularly to surround each of the plural lighting window areas with an inclination between the first surface and the lighting window areas in a cup shape.

The recess includes an annular region disposed around each of the lighting window areas. An extension region is formed to extend along the distal projection from the annular region, for introducing the washing fluid from the distal projection into the annular region.

An inner surface of the recess is continuous with a surface of the distal projection.

The recess is in an arcuate shape, and the plural lighting window areas are contained in a profile of the recess.

The distal projection is in a circumferential shape, and disposed to surround the first surface.

In one preferred embodiment, the distal projection is in an arcuate shape, and the recess is formed to extend along the distal projection.

The recess includes a second surface formed to extend substantially flatly, and having a profile containing the plural lighting window areas. A guide surface is inclined from the second surface toward the first surface with respect to the axial direction.

The distal projection includes a center portion disposed to project from the first surface at predetermined height. First and second end portions are disposed at respectively lateral sides of the center portion, and having an inclined surface inclined at a height increasing from the first surface toward the center portion.

In another preferred embodiment, furthermore, first and second fluid ports are disposed at respectively lateral ends of the distal projection, formed to retreat from the first surface by extending the recess to an outer surface of the head assembly, for exiting the washing fluid from the recess.

Furthermore, a guide surface is formed with an edge of the recess and inclined toward the first surface.

Furthermore, a second surface is formed to extend substantially flatly, and having a profile containing the plural lighting window areas. A wall projection is disposed to surround each of the lighting window areas and to project from the second surface. An inclined surface is formed on the wall projection, and inclined with a decreasing height toward the second surface.

The distal projection is in an arcuate shape, and the recess is formed to extend along the distal projection.

Consequently, the plural lighting window areas can be protected from sticking of dirt or other unwanted fluid, because the distal projection receives the washing fluid reliably and the lighting window areas can be protected by collecting the washing fluid.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an endoscope system;
Fig. 2 is a perspective view illustrating a head assembly;
Fig. 3 is a vertical section taken on line III-III in Fig. 2, illustrating the head assembly with an end nozzle device;
Fig. 4 is a vertical section taken on line IV-IV in Fig. 2, illustrating the head assembly;
Fig. 5 is a perspective view illustrating one preferred endoscope having recesses of an eccentric form;
Fig. 6 is a perspective view illustrating another preferred endoscope having an arcuate recess within which the lighting window areas are arranged;
Fig. 7 is a plan illustrating the head assembly;
Fig. 8 is a perspective view illustrating one preferred endoscope having a distal projection of a circumferential form;
Fig. 9 is a perspective view illustrating still another preferred endoscope having an arcuate recess with fluid ports;
Fig. 10 is a plan illustrating the head assembly;
Fig. 11 is a vertical section taken on line XI-XI in Figs. 9 and 10, illustrating the head assembly;
Fig. 12 is a vertical section taken on line XII-XII in Figs. 9 and 10, illustrating the head assembly.

### DETAILED DESCRIPTION OF THE PREFERRED EMBODIMENT(S) OF THE PRESENT INVENTION

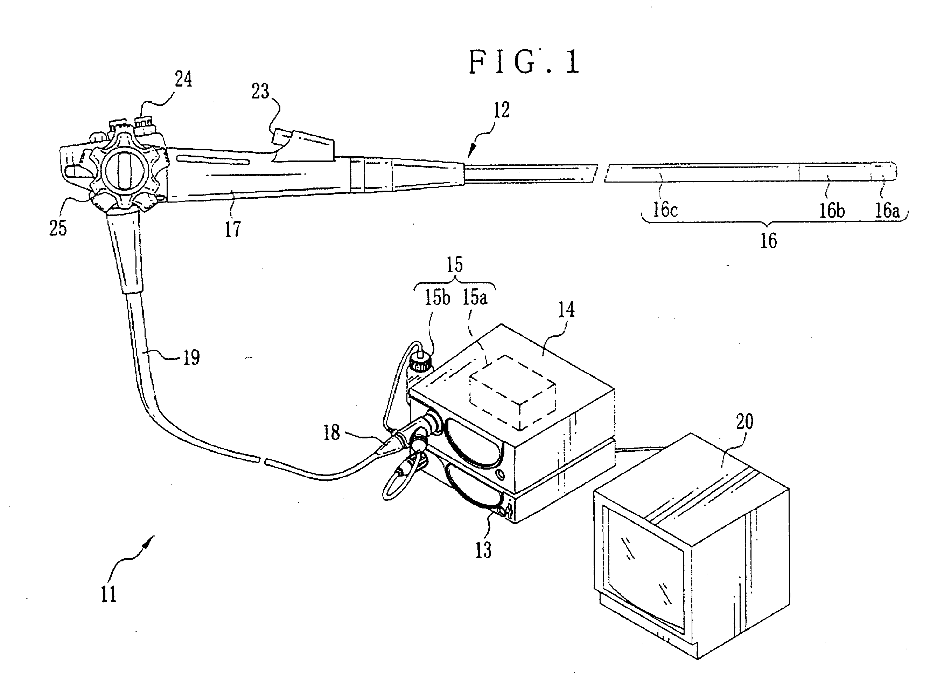

In Fig. 1, an electronic endoscope system 11 includes an electronic endoscope 12, a processing apparatus 13, a light source apparatus 14 and a fluid supply source 15. The fluid supply source 15 includes an air pump 15a for air supply, and a water reservoir 15b or a tank for washing. The air pump 15a is a well-known device, is incorporated in the light source apparatus 14, and supplies air. The water reservoir 15b is separate from the light source apparatus 14, and stores water as washing fluid. The endoscope 12 includes a section of an elongated tube 16, a handle device 17, a connector plug 18 and a universal cable 19. The elongated tube 16 is flexible and entered in a body of a patient. The handle device 17 is connected with a proximal end of the elongated tube 16. The connector plug 18 is plugged with the processing apparatus 13 and the light source apparatus 14. The universal cable 19 extends between the handle device 17 and the connector plug 18. The connector plug 18 is a composite type to which the processing apparatus 13, the light source apparatus 14 and the fluid supply source 15 are coupled.

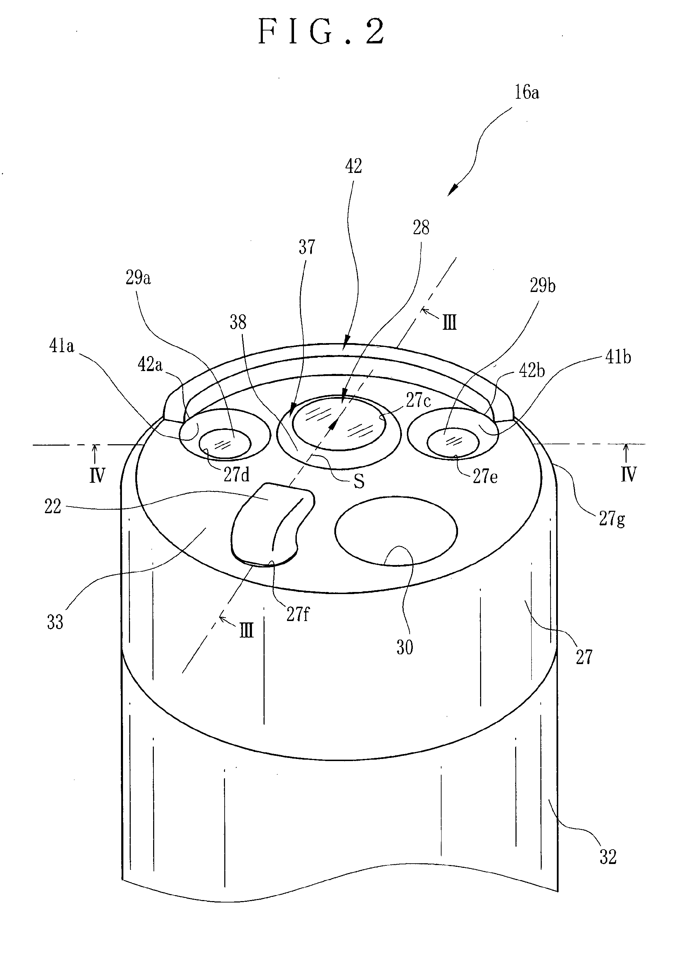

The elongated tube 16 includes a head assembly 16a, a steering device 16b, and a flexible tube 16c. A CCD 39 or image sensor of Fig. 2 is incorporated in the head assembly 16a. The steering device 16b is disposed at a proximal end of the head assembly 16a for steering. The flexible tube 16c is disposed at a proximal end of the steering device 16b.

The processing apparatus 13 is connected with the light source apparatus 14, and controls functions of the endoscope system 11. The processing apparatus 13 supplies the endoscope 12 with power through a cable extending through the universal cable 19 and the elongated tube 16, and controls the CCD 39. Also, the processing apparatus 13 receives an image signal from the CCD 39 through the cable, and creates image data by image processing of the image signal. A monitor display panel 20 is driven by means of the cable from the processing apparatus 13 to display an image according to the image data created by the processing apparatus 13.

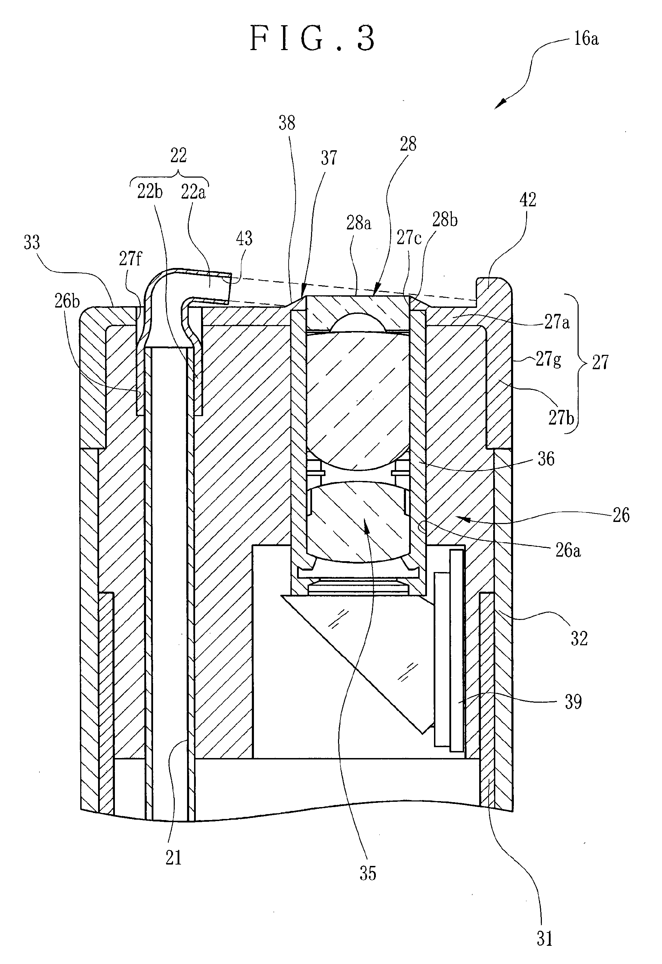

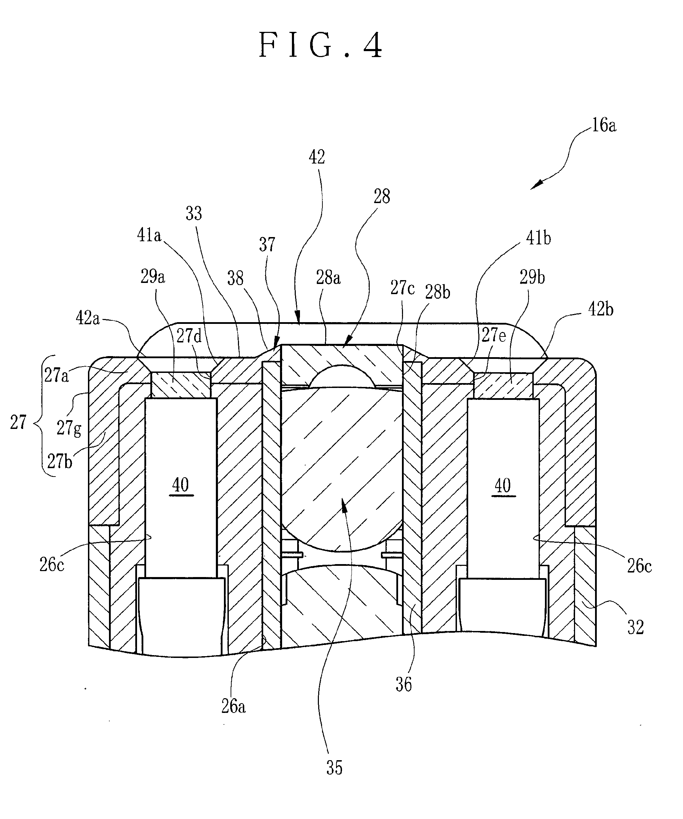

In Fig. 3, a fluid channel 21 is formed through the elongated tube 16 and the handle device 17. An end nozzle device 22 (end nozzle) is incorporated in the head assembly 16a, and has a nozzle opening with which the fluid channel 21 communicates. See Figs. 2-4. The fluid channel 21 extends through the universal cable 19 and is connected with the fluid supply source 15.

The handle device 17 includes a proximal instrument opening 23, a fluid supply button 24 and steering wheels 25. The proximal instrument opening 23 receives entry of any of various medical instruments, such as an injection needle, electrocautery device of high frequency and the like. When the fluid supply button 24 is depressed for air supply, air generated by the air pump 15a is caused to flow to the end nozzle device 22. When the fluid supply button 24 is depressed for water supply, water as washing fluid in the water reservoir 15b is caused to flow to the end nozzle device 22 by pressure of the air from the air pump 15a. The end nozzle device 22 selectively ejects air or the washing fluid from the fluid channel 21.

When the steering wheels 25 are rotated, control wires in the elongated tube 16 are moved back and forth to steer the steering device 16b up or down or to the right or left. Thus, the head assembly 16a is bent in a desired direction in a body cavity.

In Figs. 2, 3 and 4, the elongated tube 16 includes an end shell 26, a head cap 27, an imaging window area 28 or window unit, lighting window areas 29a and 29b or window units, and a distal instrument opening 30, as well as the end nozzle device 22. The end shell 26 includes mount holes 26a, 26b and 26c formed through in the axial direction of the elongated tube 16. A lens system 35 or objective lens is mounted in the mount hole 26a as will be described later. The end nozzle device 22 is mounted in the mount hole 26b. A light guide device 40 is mounted in each of the mount holes 26c. There is a link element 31 on a distal side in the steering device 16b. A proximal end of the end shell 26 is connected to the link element 31.

The head cap 27 includes a cap plate 27a and a cap skirt 27b projecting from the cap plate 27a to extend on an outer surface of the end shell 26. An outer tube 32 covers the steering device 16b, extends toward the end shell 26. A distal end of the outer tube 32 is engaged with a proximal end of the cap skirt 27b, and attached thereto by use of adhesive agent or the like. A first surface 33 or end surface (tip surface) is located on the cap plate 27a, is flat and perpendicular to an axial direction of the elongated tube 16, and is positioned on a distal side of the head assembly 16a.

The cap plate 27a has through openings 27c, 27d and 27e. The through opening 27c is disposed in a portion near to the center of the head assembly 16a as viewed in the axial direction, and is an aperture for the imaging window area 28. The through openings 27d and 27e are apertures for the lighting window areas 29a and 29b, and are arranged symmetrically with respect to the through opening 27c. A through hole 27f and the distal instrument opening 30 are formed in the first surface 33. The through hole 27f is a mount hole for the end nozzle device 22.

The imaging window area 28 is constituted by a window lens or objective lens as an element of the lens system 35 on the distal side, and also functions as a cover of glass. The window lens is in a circular shape, and has a window surface 28a and a peripheral surface 28b around the window surface 28a.

A lens barrel 36 includes the lens system 35 as optical system aligned with the imaging window area 28. The lens barrel 36 is engaged with a proximal edge of the peripheral surface 28b of the imaging window area 28. A distal edge of the peripheral surface 28b of the imaging window area 28 is engaged with the through opening 27c of the head cap 27. The lens barrel 36 is fitted in the mount hole 26a of the end shell 26. A distal surface of the lens barrel 36 tightly contacts the cap plate 27a of the head cap 27.

The imaging window area 28 is disposed to set the window surface 28a at a predetermined height from the first surface 33. An annular projection 37 is formed on the head cap 27, is disposed between the edge of the window surface 28a and the first surface 33, and projects at a predetermined height from the first surface 33. The annular projection 37 has an inner surface where the through opening 27c is defined. An inclined surface 38 is formed around the annular projection 37, and has a height gradually decreasing from the window surface 28a toward the first surface 33.

The CCD 39 is disposed on a proximal side of the lens system 35. An example of the CCD 39 is an interline transfer CCD. An object image is focused on an imaging surface of the CCD 39 according to object light entered through the lens system 35. Note that an image sensor can be a CMOS image sensor in place of the CCD 39.

The lighting window areas 29a and 29b are constituted by window lenses, and emit light from the light source apparatus 14 toward an object of interest in a body cavity. A distal surface of the lighting window areas 29a and 29b is positioned lower than the first surface 33 in the proximal direction. An exit end of the light guide device 40 is opposed to a proximal surface of the lighting window areas 29a and 29b. The light guide device 40 includes a bundle of plural optical fibers, a ring and a tube material. The ring is fitted on a distal end of the bundled optical fibers. The tube material is fitted on the periphery of the bundled optical fibers for covering. The light guide device 40 extends through the elongated tube 16, the handle device 17, the universal cable 19 and the connector plug 18, and guides light from the light source apparatus 14 to the lighting window areas 29a and 29b. An instrument channel (not shown) is formed through the elongated tube 16. The distal instrument opening 30 is connected with the instrument channel, which communicates with the proximal instrument opening 23 in the handle device 17. A distal end of any of various medical instruments entered in the proximal instrument opening 23 appears through the distal instrument opening 30.

The cap plate 27a has recesses 41a and 41b and a distal projection 42 or fence wall. The recesses 41a and 41b are disposed around respectively the lighting window areas 29a and 29b and at a lower level than the first surface 33. The distal projection 42 projects from the first surface 33 in the distal direction. The recesses 41a and 41b have a circular profile, and are inclined from the first surface 33 toward the lighting window areas 29a and 29b conically in a cup shape.

The end nozzle device 22 is one piece including a nozzle spout 22a with a spout channel, and a flow conduit 22b or coupling sleeve. The flow conduit 22b is coupled to a distal end of the fluid channel 21. The flow conduit 22b and the fluid channel 21 are fitted in the mount hole 26b of the end shell 26. A nozzle opening 43 is defined in the nozzle spout 22a. The nozzle spout 22a is in a gradually curved shape from the flow conduit 22b toward its end, and appears externally through the through hole 27f of the head cap 27. A region of ejecting fluid from the end nozzle device 22 is so predetermined as to blow the fluid at least to the inclined surface 38, and preferably so predetermined as to blow the fluid to both the imaging window area 28 and the inclined surface 38.

The distal projection 42 is disposed on a side in a path direction S from the end nozzle device 22, and projects in a distal direction. The fluid, when ejected from the end nozzle device 22, moves past the inclined surface 38 and the imaging window area 28, and then returns upon impingement with the distal projection 42. The head cap 27 has an outer surface 27g. The distal projection 42 projects from the first surface 33, and is in an arcuate shape along an edge of the head assembly 16a with the outer surface 27g. Also, the distal projection 42 extends to positions near to the lighting window areas 29a and 29b. End portions 42a and 42b of the distal projection 42 are formed to extend to the recesses 41a and 41b in a directly continuous manner of their surfaces.

The operation of washing the imaging window area 28 with the washing fluid from the end nozzle device 22 (end nozzle) in the endoscope 12 is described now as a process of the invention. The washing fluid such as air or water is ejected by the end nozzle device 22 and partially impinges on the inclined surface 38. The washing fluid on the inclined surface 38 is spread in the radial direction of the imaging window area 28 and moves up on the inclined surface 38. The washing fluid extends to the entirety of the window surface 28a of the imaging window area 28 to blow away body fluid or dirt from the window surface 28a. Also, air is blown to remove the water or liquid as washing fluid.

A component of washing fluid moved past the imaging window area 28 and the inclined surface 38, among components ejected by the end nozzle device 22, impinges on and is returned by the distal projection 42 to the center of the head assembly 16a. As the recesses 41a and 41b are nearer to the center than the distal projection 42, the returned washing fluid enters the recesses 41a and 41b. The returned washing fluid remains in the recesses 41a and 41b on the window surface of the lighting window areas 29a and 29b owing to the surface tension of the liquid.

Consequently, the imaging window area 28 can be washed by the washing fluid from the end nozzle device 22. The washing fluid remains in the recesses 41a and 41b. It is possible to protect the lighting window areas 29a and 29b by use of the washing fluid remaining in the recesses 41a and 41b, to prevent deposition of body fluid or dirt thereon. Although not washed with the washing fluid from the end nozzle device 22, the lighting window areas 29a and 29b are protected by the washing fluid. It is possible to prevent drop in a light amount of the light from the lighting window areas 29a and 29b.

As the recesses 41a and 41b are formed in the cup shape, washing fluid can enter those easily. The washing fluid returned by the distal projection 42 is introduced in the recesses 41a and 41b, because the distal projection 42 has the surface extending continuously with inner surfaces of the recesses 41a and 41b.

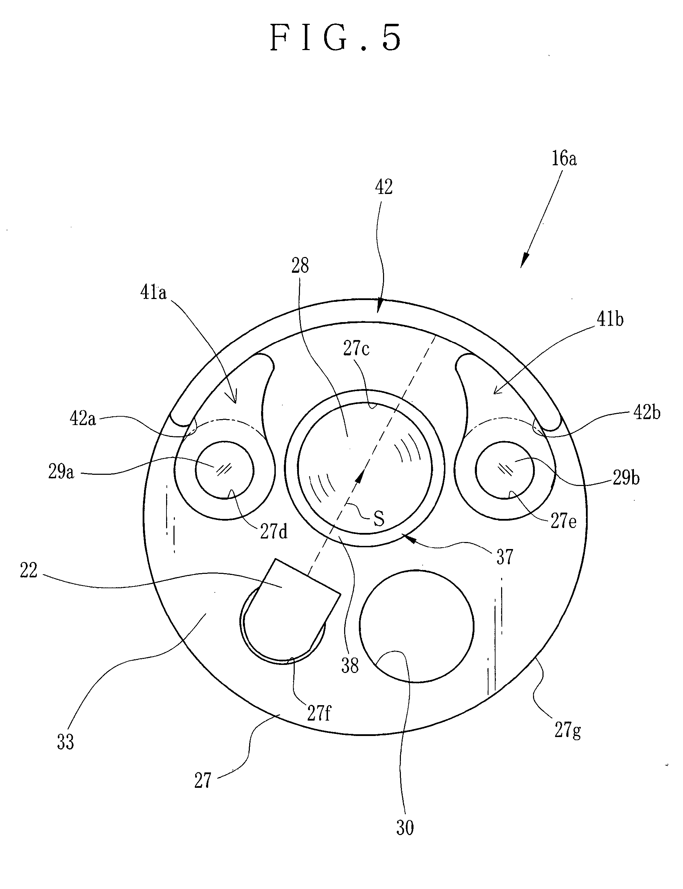

In Fig. 5, another preferred embodiment is illustrated, in which the recesses 41a and 41b are inclined toward the lighting window areas 29a and 29b from a profile of an eccentrically extending shape (such as a wedge shape) extending toward the distal projection 42, inclusive of an annular region and a triangular extension region which are indicated with the phantom line. The extension region makes it easier to direct the returned washing fluid from the distal projection 42 into the recesses 41a and 41b. The end portions 42a and 42b of the distal projection 42 are preferably disposed to extend to the recesses 41a and 41b in a continuous manner of their surfaces. In the above embodiment, the lighting window areas are two in the head assembly 16a. However, three or more lighting window areas can be formed in the head assembly 16a. In combination with those, a recess can be formed in the head assembly with a profile containing the lighting window areas, and retreat in the proximal direction from the first surface 33, in the form similar to the recesses 41a and 41b.

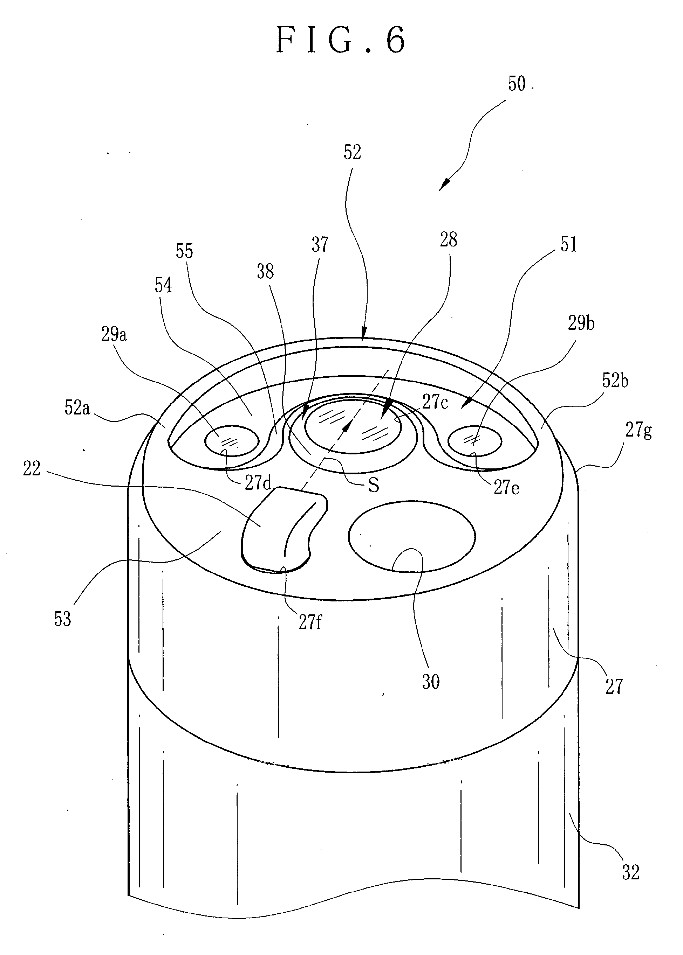

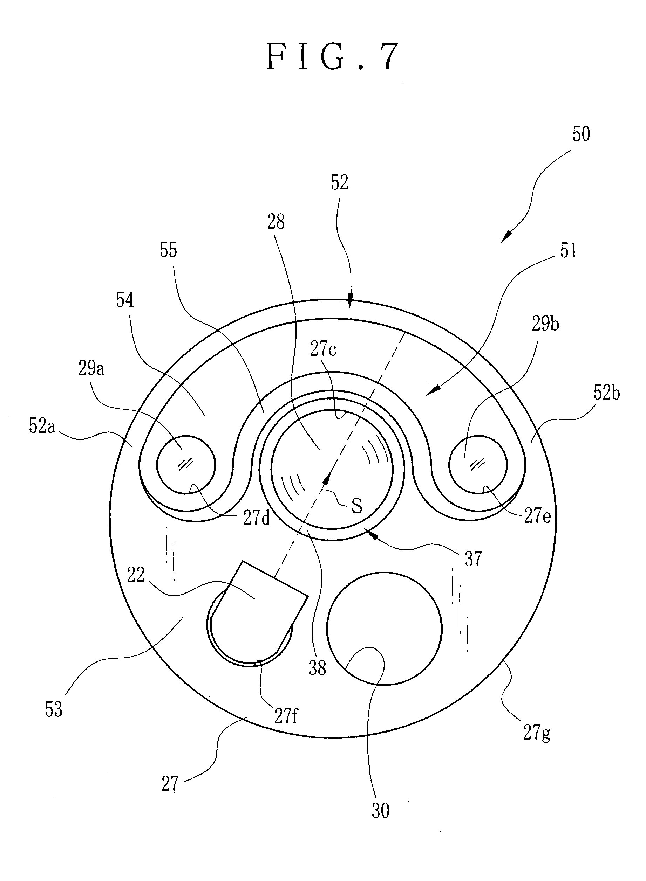

A recess may be formed in a different manner from the recesses 41a and 41b around the lighting window areas 29a and 29b of the above embodiment. In Figs. 6 and 7, another preferred head assembly 50 is illustrated. A recess 51 is formed in the head assembly 50 and disposed with a profile containing the lighting window areas 29a and 29b. A distal projection 52 or fence wall projects from the head assembly 50, and is disposed in the path direction S of the end nozzle device 22. Elements similar to those of the above embodiment are designated with identical reference numerals.

The head assembly 50 has a first surface 53 or end surface (tip surface). The recess 51 retreats in the proximal direction from the first surface 53, is defined by two arcs between which the lighting window areas 29a and 29b are disposed, and is disposed on a side of the path direction S of the end nozzle device 22. The recess 51 has a second surface 54 (recess surface) and a guide surface 55. The second surface 54 is flat and retreats from the first surface 53 in the proximal direction. The guide surface 55 is inclined from the periphery of the imaging window area 28 toward the second surface 54 in the proximal direction. The first surface 53 is perpendicular to the axial direction of the elongated tube 16 in a similar manner to the first surface 33 of the first embodiment, and is positioned on a distal side in the head assembly 50. The lighting window areas 29a and 29b are set flush with the second surface 54. Note that the second surface 54 can be preferably oriented in parallel with the first surface 53.

The distal projection 52 projects in the distal direction from the second surface 54 and disposed along the outer surface 27g. First and second end portions 52a and 52b of the distal projection 52 are formed to extend continuously with the first surface 53. Thus, washing fluid can be easily introduced in the recess 51 which is surrounded by the first surface 53 and the distal projection 52.

This being so, the washing fluid, returned by the distal projection 52 or moved past the imaging window area 28, comes to remain in the recess 51 after ejection from the end nozzle device, because the recess 51 is formed in the head assembly 50. The washing fluid, because guided by the guide surface 55, remains on the window surface of the lighting window areas 29a and 29b. Accordingly, it is possible to protect the lighting window areas 29a and 29b by use of the washing fluid remaining in the recess 51, to prevent deposition of body fluid or dirt thereon.

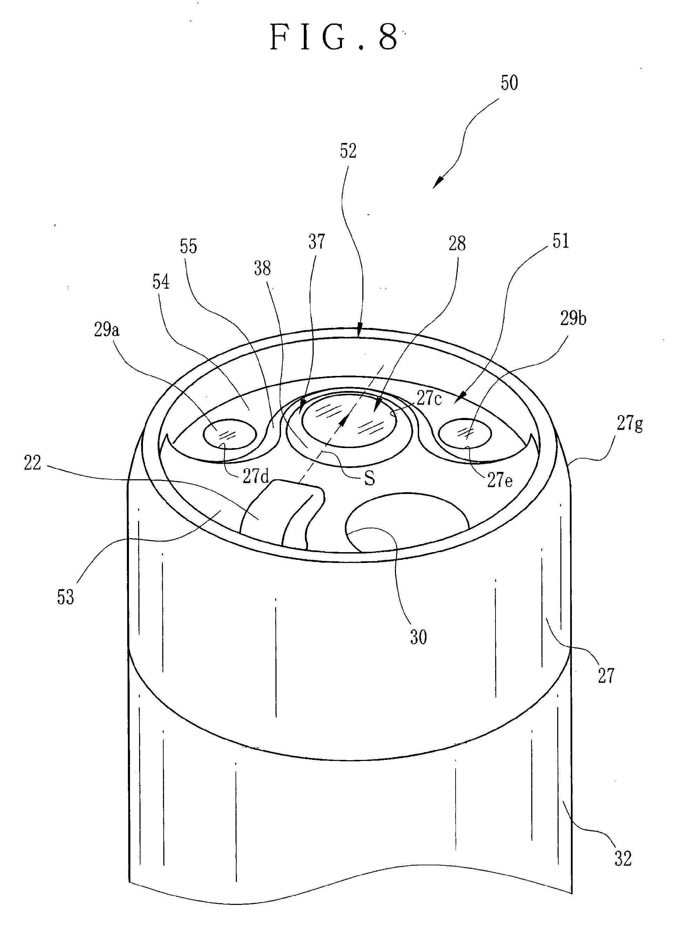

In the present embodiment, the distal projection 52 projects from the second surface 54, and has the first and second end portions 52a and 52b having surfaces continuous with the first surface 53. In Fig. 8, still another preferred embodiment is illustrated, in which the distal projection 52 extends circumferentially at the head assembly 50, and projects from the first surface 53 and the second surface 54 in a distal direction. In the above embodiment, the lighting window areas are two in the head assembly 50. However, the head assembly 50 may have three or more lighting window areas. For this structure, a single recess similar to the recess 51 in the above embodiment can be formed to retreat in the proximal direction from the first surface 53 and can be disposed with a profile containing the plural light window areas.

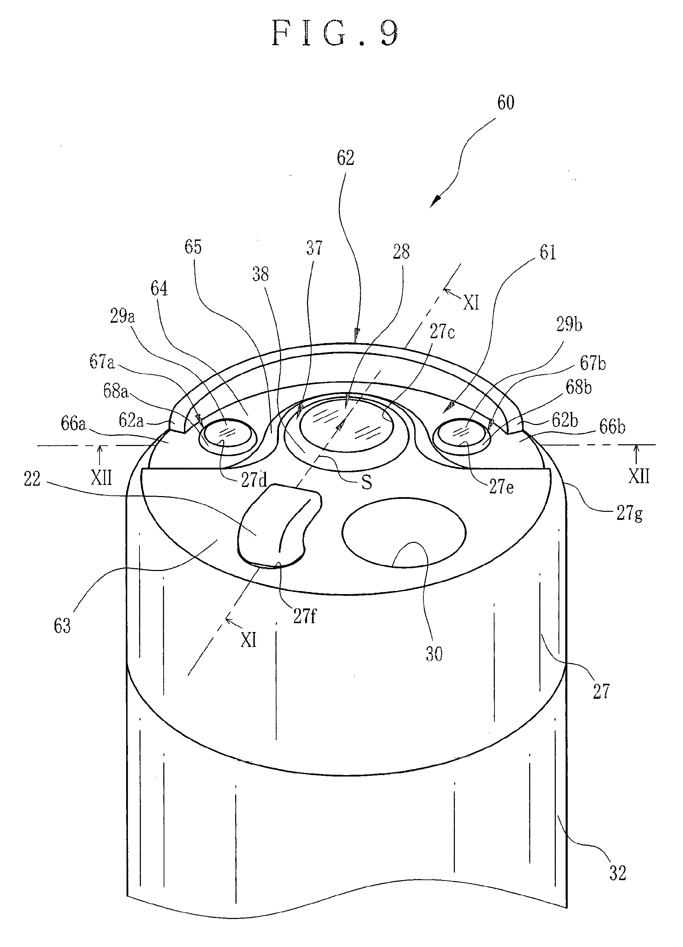

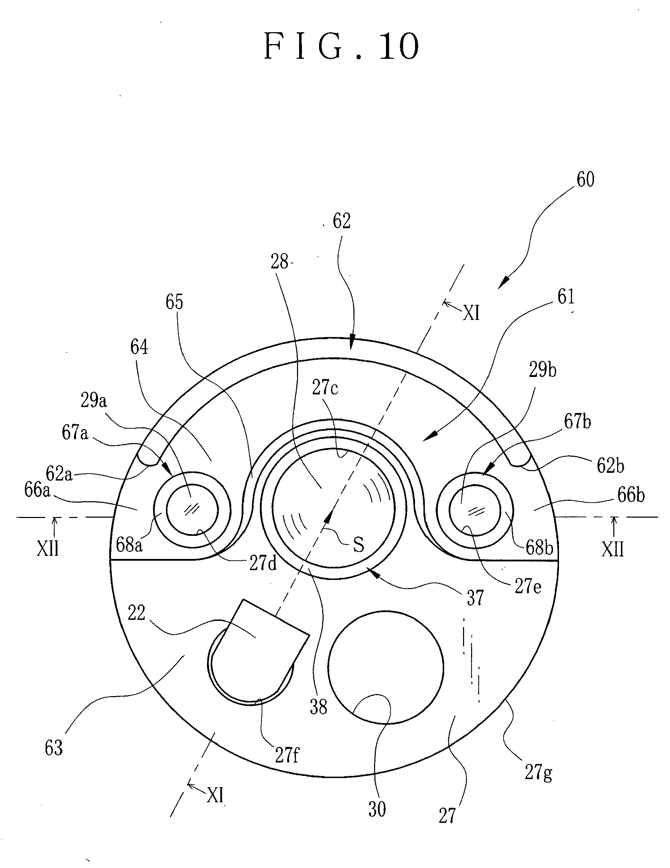

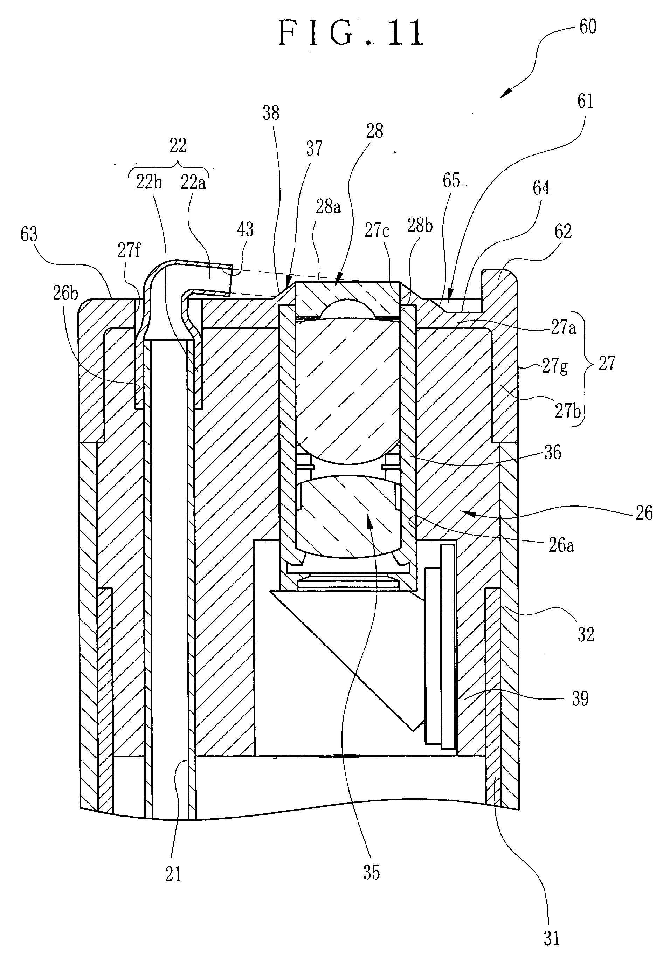

In the above embodiment, the washing fluid is caused to remain in the recesses 41a and 41b or the recess 51. In Figs. 9-12, another preferred head assembly 60 is illustrated. A recess 61 is formed in the head assembly 60 with a profile containing the lighting window areas 29a and 29b, and extends laterally to the outer surface 27g of the head assembly 60. A distal projection 62 or fence wall is formed on a side of the path direction of the end nozzle device 22, to cause the washing fluid to flow from the recess 61 to the outside.

The head assembly 60 has a first surface 63 or end surface. The recess 61 retreats in the proximal direction from the first surface 63, and is disposed on a side of the path direction S of the end nozzle device 22. The recess 61 has a second surface 64 (recess surface) and a guide surface 65. The second surface 64 has a profile containing the lighting window areas 29a and 29b, and retreats from the first surface 63 in the proximal direction. The guide surface 65 is inclined from the periphery of the imaging window area 28 toward the second surface 64 in the proximal direction. The first surface 63 is perpendicular to the axial direction of the elongated tube 16 in a similar manner to the first surface 33 of the first embodiment, and is positioned on a distal side in the head assembly 60. Note that the second surface 64 can be preferably oriented in parallel with the first surface 63.

The distal projection 62 projects from the second surface 64 in the distal direction, extends along the outer surface 27g, and is arcuate to extend toward the lighting window areas 29a and 29b. The distal projection 62 has end portions 62a and 62b arranged equidistantly from the area of the first surface 63. First and second fluid ports 66a and 66b are defined between the first surface 63 and the end portions 62a and 62b of the distal projection 62, for discharge of washing fluid between the second surface 64 and the outer surface 27g.

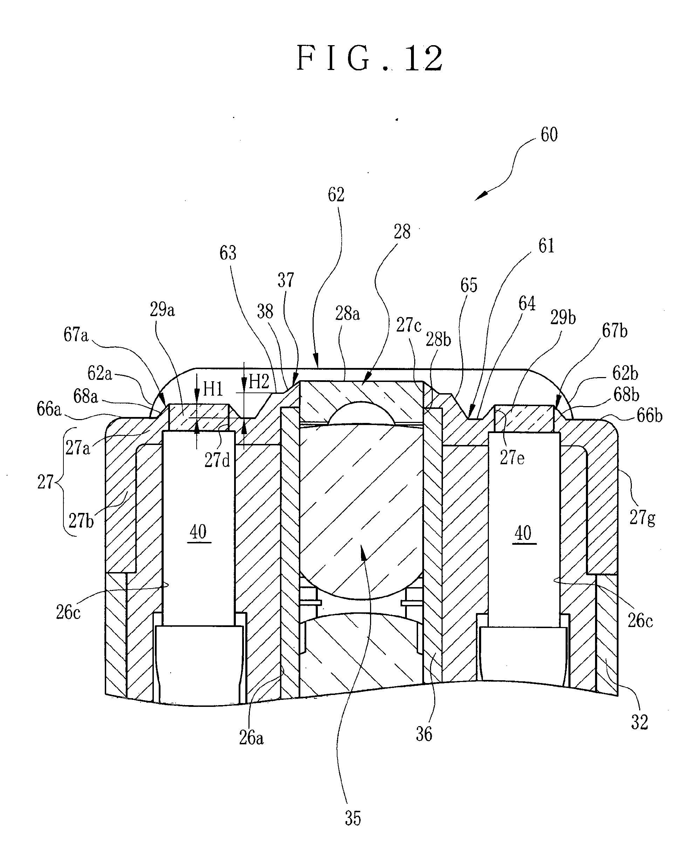

Wall projections 67a and 67b or peripheral projections are formed in the head assembly 60, project from the second surface 64 in the distal direction, and are disposed around the lighting window areas 29a and 29b. Inclined surfaces 68a and 68b are disposed on the wall projections 67a and 67b. The inclined surfaces 68a and 68b are inclined from the lighting window areas 29a and 29b toward the second surface 64 in the proximal direction. Note that window surfaces of the lighting window areas 29a and 29b are set flush with a distal end of the wall projections 67a and 67b. A height H1 of the window surfaces (see Fig. 12) is predetermined lower than a height H2 of the first surface 63 with reference to the second surface 64. Thus, drainage of washing fluid on the surfaces of the lighting window areas 29a and 29b can be effective considerably.

Thus, the washing fluid, returned by the distal projection 62 or blown past the imaging window area 28, passes the first and second fluid ports 66a and 66b to flow out after ejection from the end nozzle device 22, because of the recess 61 and positions of the lighting window areas 29a and 29b lower than the first surface 63. Also, the guide surface 65 causes the washing fluid to flow on the window surface of the lighting window areas 29a and 29b. Accordingly, it is possible to prevent deposition of body fluid or dirt on the window surface of the lighting window areas 29a and 29b. Such body fluid or dirt can be eliminated by washing with the washing fluid.

In the present embodiment, the lighting window areas are two in the head assembly 60. However, the head assembly 60 may have three or more lighting window areas. For this structure, a single recess similar to the recess 61 in the embodiment can be formed to retreat in the proximal direction from the first surface 63, and can have a profile containing the plural light window areas to extend laterally to the outer surface 27g of the head assembly 60 through the fluid ports.

In the above embodiment, the distal window surface of the imaging window area 28 is flat. However, the window surface may be convex. A peripheral edge portion of the convex window surface may be flush with the distal end of the inclined surface 38, or may project from the distal end of the inclined surface 38 in the distal direction. Also, the imaging window area 28 may be disposed to keep the window surface 28a flush with the first surface without forming the annular projection 37 thereabout. Furthermore, the imaging window area 28 may project from the first surface with a certain height. An edge portion of the imaging window area 28 can be formed with inclination in a projecting form from the first surface without forming the annular projection 37.

Note that the first surface 33, 53, 63 may be inclined slightly with reference to a radial direction of the head assembly 16a. Also, the first surface may be a slightly curved surface.

In the embodiments of the drawings except for Fig. 8, the distal projection 42, 52, 62 is arcuate in a size being approximately 1/3 as long as the circumference. However, the distal projection may have other sizes, for example, can have a form of a semicircle, a one fourth of the circumference, and the like. Furthermore, the distal projection may be constituted only by flat surfaces, namely can be a wall of a composite shape obtained by connecting plural flat surfaces along the periphery of the first surface.

In the above embodiments, the recesses 41a, 41b, 51, 61 extend to come around each of the lighting window areas 29a and 29b. However, it is possible according to the feature of the invention that at least one portion of the peripheral edge of the lighting window areas is directly continuous with the recess. Also, the recess can be formed with a decreasing width, and can be formed in a groove shape, funnel shape and the like. The recesses 41a and 41b, although circular according to the embodiment, can be in a shape of an eccentric circle, crescent shape, elliptical shape, polygonal shape and the like.

In the above embodiments, the head cap 27 is originally separate from the end shell 26. However, the end shell can be one piece including the head cap formed therewith.

In the above embodiments, the endoscope includes the solid state imaging unit. However, the present invention can be used in an endoscope in which an optical image guide device is used for imaging. In the above embodiments, the washing fluid is water. However, various examples of washing fluid available in the field of the endoscope can be used, for example, alcohol, a mixture of water with fine particles for washing, and the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope having a section of an elongated tube (16) for entry in a body cavity, comprising:
a first surface (33, 53, 63), formed at a distal end of a head assembly (16a, 50, 60) included in said elongated tube, and disposed to extend crosswise to an axial direction of said elongated tube;
an imaging window area (28), disposed in said first surface, for receiving light from said object;
an end nozzle (22), disposed on said first surface, for washing said imaging window area by ejecting washing fluid thereto;
a distal projection (42, 52, 62), formed on said head assembly at a higher level than said first surface, for receiving said washing fluid from said imaging window area; **characterized in** further comprising a recess (41a, 41b, 51, 61) formed in said head assembly at a lower level than said first surface; and
at least one lighting window area (29a, 29b), disposed in said recess, for applying light to an object in said body cavity.

2. An endoscope as defined in claim 1, **characterized in that** a surface height of said imaging window area is larger than a height of said first surface.

3. An endoscope as defined in claim 1, **characterized in that** said recess is annular and tapered, and disposed to surround said lighting window area.

4. An endoscope as defined in claim 1, **characterized in that** said recess includes:
an annular region formed in a cup shape, and disposed to surround said lighting window area;
an extension region, disposed to extend from said lighting window area toward said distal projection, for introducing said washing fluid from said distal projection into said annular region.

5. An endoscope as defined in claim 3 or 4, **characterized in that** said recess extends continuously with said distal projection.

6. An endoscope as defined in claim 1, **characterized in that** said distal projection extends at least partially along an outer surface of said head assembly;
said recess is disposed between a periphery of said imaging window area and said distal projection, is in an arcuate shape, and has a second surface where said lighting window area is disposed.

7. An endoscope as defined in claim 6, **characterized in** further comprising an inclined guide surface disposed to extend continuously between said first and second surfaces.

8. An endoscope as defined in claim 6, **characterized in that** said distal projection is in an arcuate shape, and has a center portion and two end portions.

9. An endoscope as defined in claim 8, **characterized in that** said center portion has a large height in said distal projection, and said end portions are inclined with a height increasing from said first surface to said center portion.

10. An endoscope as defined in claim 8, **characterized in** further comprising first and second fluid ports, disposed between said first surface and said end portions to extend from said recess to said outer surface of said head assembly, for directing said washing fluid.

11. An endoscope as defined in claim 7, **characterized in that** said guide surface is disposed along a periphery of said imaging window area.

12. An endoscope as defined in any one of claims 6-11, **characterized in** further comprising a wall projection, formed to project from said second surface, disposed to surround said lighting window area, and inclined with respect to said axial direction toward said second surface.

## Patentansprüche

1. Ein Endoskop mit einem Abschnitt in Form eines länglichen Schlauchs (16) zum Einführen in einen Körperhohlraum, umfassend:
eine erste Fläche (33, 53, 63), die an einem distalen Ende einer in dem länglichen Schlauch enthaltenen Kopfanordnung (16a, 50, 60) enthalten ist und sich quer zu einer axialen Richtung des länglichen Schlauches erstreckt;
einen Abbildungsfensterbereich (28), die in der ersten Fläche gelegen ist, um Licht von dem Objekt zu empfangen;
eine Enddüse (22), die an der ersten Fläche gelegen ist, um den Abbildungsfensterbereich durch Ausstoßen eines Spülfluids auf den Bereich zu spülen;
einen distalen Vorsprung (42, 52, 62), der an der Kopfanordnung in einer höheren Ebene als die erste Fläche ausgebildet ist, um das Spülfluid von dem Abbildungsfensterbereich aufzunehmen, **gekennzeichnet durch** weiterhin:
eine in der Kopfanordung in einer niedrigeren Ebene als die erste Fläche ausgebildete Ausnehmung (41a, 41b, 51, 61); und
mindestens einen in der Ausnehmung befindlichen Beleuchtungsfensterbereich (29a, 29b) zum Aufbringen von Licht auf ein in dem Körperhohlraum befindliches Objekt.

2. Endoskop nach Anspruch 1, **dadurch gekennzeichnet, dass** die Oberflächenhöhe des Abbildungsfensterbereiches größer ist als eine Höhe der ersten Fläche.

3. Endoskop nach Anspruch 1, **dadurch gekennzeichnet, dass** die Ausnehmung kreisförmig und tonisch ist und derart angeordnet ist, dass sie den Beleuchtungsfensterbereich umfasst.

4. Endoskop nach Anspruch 1, **dadurch gekennzeichnet, dass** die Ausnehmung enthält:
eine becherförmige ringförmige Zone, die so gelegen ist, dass sie den Beleuchtungsfensterbereich umfasst;
eine Erweiterungszone, angeordnet zur Erstreckung von dem Beleuchtungsfensterbereich in Richtung des distalen Vorsprungs, um das Spülfluid aus dem distalen Vorsprung in die ringförmige Zone einzubringen.

5. Endoskop nach Anspruch 3 oder 4, **dadurch gekennzeichnet, dass** die Ausnehmung sich kontinuierlich anschließend an den distalen Vorsprung erstreckt.

6. Endoskop nach Anspruch 1, **dadurch gekennzeichnet, dass** der distale Vorsprung sich zumindest teilweise entlang einer Außenfläche der Kopfanordnung erstreckt;
die Ausnehmung sich zwischen einem Umfang des Abbildungsfensterbereichs und dem distalen Vorsprung befindet, eine bogenförmige Gestalt aufweist und eine zweite Fläche besitzt, wo sich der Beleuchtungsfensterbereich befindet.

7. Endoskop nach Anspruch 6, **dadurch gekennzeichnet, dass** es außerdem eine geneigte Führungsfläche besitzt, die sich kontinuierlich zwischen der ersten und der zweiten Fläche erstreckt.

8. Endoskop nach Anspruch 6, **dadurch gekennzeichnet, dass** der distale Vorsprung eine bogenförmige Gestalt aufweist und einen Mittelbereich sowie zwei Endbereiche besitzt.

9. Endoskop nach Anspruch 8, **dadurch gekennzeichnet, dass** der Mittelbereich eine große Höhe in dem distalen Vorsprung aufweist, und die Endbereiche geneigt sind mit einer sich ausgehend von der ersten Fläche zu dem Mittelbereich zunehmenden Höhe.

10. Endoskop nach Anspruch 8, **dadurch gekennzeichnet, dass** es außerdem eine erste und eine zweite Fluidöffnung aufweist, angeordnet zwischen der ersten Fläche und den Endbereichen, um sich ausgehend von der Ausnehmung zu der Außenfläche der Kopfanordnung erstreckt, um das Spülfluid zu lenken.

11. Endoskop nach Anspruch 7, **dadurch gekennzeichnet, dass** die Führungsfläche sich entlang einer Peripherie des Abbildungsfensterbereichs erstreckt.

12. Endoskop nach einem der Ansprüche 6 bis 11, **dadurch gekennzeichnet, dass** es weiterhin einen Wandvorsprung aufweist, der von der zweiten Fläche vorspringt, angeordnet ist zum Umfassen des Beleuchtungsfensterbereichs und in Bezug auf die axiale Richtung zu der zweiten Fläche hin geneigt ist.

## Revendications

1. Endoscope ayant une section d'un tube allongé (16) pour entrer dans une cavité corporelle, comprenant:
une première surface (33, 53, 63) formée au niveau d'une extrémité distale d'un ensemble de tête (16a, 50, 60) inclus dans ledit tube allongé, et disposée pour s'étendre en diagonale par rapport à une direction axiale dudit tube allongé;
une zone de fenêtre d'imagerie (28), disposée dans ladite première surface, pour recevoir la lumière dudit objet;
une buse d'extrémité (22) disposée sur ladite première surface, pour laver ladite zone de fenêtre d'imagerie en éjectant sur cette dernière du fluide de lavage;
une saillie distale (42, 52, 62) formée sur ledit ensemble de tête à un niveau plus haut que ladite première surface, pour recevoir ledit fluide de lavage de ladite zone de fenêtre d'imagerie; **caractérisé en ce qu'**il comprend en outre:
un évidement (41a, 41b, 51, 61) formé dans ledit ensemble de tête à un niveau plus bas que ladite première surface; et
au moins une zone de fenêtre d'éclairage (29a, 29b) disposée dans ledit évidement, pour appliquer de la lumière sur un objet dans ladite cavité corporelle.

2. Endoscope selon la revendication 1, **caractérisé en ce qu'**une hauteur de surface de ladite zone de fenêtre d'imagerie est supérieure à une hauteur de ladite première surface.

3. Endoscope selon la revendication 1, **caractérisé en ce que** ledit évidement est annulaire et progressivement rétréci, et disposé pour entourer ladite zone de fenêtre d'éclairage.

4. Endoscope selon la revendication 1, **caractérisé en ce que** ledit évidement comprend:
une région annulaire formée en une forme de coupe, et disposée pour entourer ladite zone de fenêtre d'éclairage;
une région d'extension, disposée pour s'étendre de ladite zone de fenêtre d'éclairage vers ladite saillie distale, pour introduire ledit fluide de lavage de ladite saillie distale dans ladite région annulaire.

5. Endoscope selon la revendication 3 ou 4, **caractérisé en ce que** ledit évidement s'étend de manière continue avec ladite saillie distale.

6. Endoscope selon la revendication 1, **caractérisé en ce que** ladite saillie distale s'étend au moins partiellement le long d'une surface externe dudit ensemble de tête;
ledit évidement est disposé entre une périphérie de ladite zone de fenêtre d'imagerie et ladite saillie distale, a une forme arquée, et a une seconde surface dans laquelle ladite zone de fenêtre d'éclairage est disposée.

7. Endoscope selon la revendication 6, **caractérisé en ce qu'**il comprend en outre une surface de guidage inclinée disposée pour s'étendre de manière continue entre lesdites première et seconde surfaces.

8. Endoscope selon la revendication 6, **caractérisé en ce que** ladite saillie distale est de forme arquée, et a une partie centrale et deux parties d'extrémité.

9. Endoscope selon la revendication 8, **caractérisé en ce que** ladite partie centrale a une grande hauteur dans ladite saillie distale, et lesdites parties d'extrémité sont inclinées avec une hauteur qui augmente de ladite première surface à ladite partie centrale.

10. Endoscope selon la revendication 8, **caractérisé en ce qu'**il comprend en outre des premier et second orifices de fluide, disposés entre ladite première surface et lesdites parties d'extrémité pour s'étendre dudit évidement à ladite surface externe dudit ensemble de tête, afin de diriger ledit fluide de lavage.

11. Endoscope selon la revendication 7, **caractérisé en ce que** ladite surface de guidage est disposée le long d'une périphérie de ladite zone de fenêtre d'imagerie.

12. Endoscope selon l'une quelconque des revendications 6 à 11, **caractérisé en ce qu'**il comprend en outre une saillie de paroi, formée pour faire saillie de ladite seconde surface, disposée pour entourer ladite zone de fenêtre d'éclairage, et inclinée par rapport à ladite direction axiale vers ladite seconde surface.
